# EUROPEAN PATENT APPLICATION

(11) **EP 2 638 892 A1**
(43) Date of publication of application: **18.09.2013**
(21) Application number: 12001705.8
(22) Date of filing: 13.03.2012
(51) Int. Cl.: A61K 6/06, A61K 6/02, A61L 24/00, A61L 27/00

(54) **Bioactive hydraulic organic matrix materials**

(71) Applicant: S & C Polymer Silicon- und Composite-Spezialitäten GmbH, 25335 Elmshorn (DE)
(72) Inventor: Akinmade, Ade, 25335 Elmshorn (DE); Engelbrecht, Jürgen, 25335 Elmshorn (DE)
(74) Representative: Forstmeyer, Dietmar

(57) **Abstract**

The present invention is directed to novel bioactive Hydraulic Organic Matrix Materials for use in the biomedical, dental and especially the dental endodontic field.

## Description

### FIELD OF INVENTION

The present invention is directed to a bioactive Hydraulic Organic Matrix Material (HOMM) for use in the biomedical and dental fields, especially the in the dental endodontic field. The present invention provides a water-setting material of the type Hydraulic Organic Matrix Material that can be preferably used as a formable pasty water-hardening direct restorative material, especially as a root canal filling material in the dental endodontic field. Moreover, the present invention provides pre-formed rigid shapes such as endodontic Points that are formed from the inventive Hydraulic Organic Matrix Material. Further, the present invention provides a kit comprising the inventive Hydraulic Organic Matrix Material in form of rigid pre-formed shapes and in form of a pasty water-hardening restorative.

### BACKGROUND OF THE INVENTION

The dental field of endodontics deals with the internal portion of the teeth comprising the corona chamber, root canal and periapical regions of a tooth. Endodontic filling cements are used to treat appropriate clinical conditions of these three regions. There are a number of clinical cases that necessitate the use of endodontic root canal cements. These include caries leading to irritation of the pulp by micro-organisms that ingress either from the bloodstream or from the oral environment and periodontal diseases that lead to pulpal irritation requiring dental procedures. Other cases include injury to the pulp as a result of trauma, abrasion or attrition, damage to the pulp as a result of erosion of tooth substance by acidic liquids, or pulpal damage of mechanical, thermal or chemical origin. Examples of mechanical causes of pulpal damage include i) by rotary instruments during the preparation of cavities in tooth, or ii) the insertion of dental retention pins/posts. Thermal causes usually occur from friction from high-speed rotary instruments, while chemical causes can occur when inappropriate dental restoratives (e.g. low acidity zinc phosphate cements or acid etching) are placed on the dentinal floor in direct contact to the pulp. Therefore, endodontic filling cements are used in dental procedures of the above-outlined cases in four broad categories: i) as root apex end sealer, ii) as root canal filling material in Pulpectomy, iii) as root dentine wall repair material, and iv) as pulp capping cement.

In all of these applications, as a minimum, endodontic root canal cements must be insoluble in oral fluids and be dimensionally stable. Ideally, they should be biocompatible or better still, bioactive and possess good sealing properties in the restored teeth.

In addition to the outlined endodontic field, there is sometimes a need to apply a temporary restoration in a dental cavity, for example in the interim while a permanent indirect restoration is being prepared. Such a temporary restoration will typically be of intermediate strength and primarily serve the function of sealing the prepared dental cavity from bacterial ingress. The material of choice for such applications therefore is an easy to use material with good sealing properties.

### DEVELOPMENTS IN THE FIELD OF DENTAL PROPHYLAXIS AND PASTES

Georg Dietz in the US Patent No. 4311528 of 1982 disclosed a root filling paste for dental surgery containing calcium hydroxide and oleum pedum tauri (neatsfoot oil), a naturally-occurring oil formed from a mixture of fatty acids and glycerol.

Rainer Lueck and Swen Neander in the patent US 2010/0068305 A1 of 2010 describe shelf-stable mixture comprising at least one metal hydroxide and wax esters as a potential wound dressing dental material.

Both the above mentioned patents relate to and describe non-hardening soft pasty direct restorative materials for use in the dental environment to be applied to inflamed tissue, for example in combating periapical pain following endodontic root canal surgery.

### PROBLEMS OF THE STATE-OF-THE-ART OF DENTAL ENDODONTIC MATERIALS

Thermoplastic materials have been used in the dental field for many decades. In the dental endodontic field, the most famous being Gutta Percha Points. Gutta Percha Points are typically used in the dental endodontic field as so-termed "indirect filling materials". The term "indirect filling material" refers to a material which is pre-formed and then applied or bonded into place in the prepared oral environment. In contrast thereto, the term "direct filling material" refers to a material which is placed in the oral environment while fluid and thus allowed to take the shape of the prepared cavity before hardened in situ. Since the primary requirement of a root canal filling material is to completely obliterate, i.e. fill the canal as far as the apical limit, shaped and calibrated Gutta Percha Points are usually selected for this purpose with the pre-requisite that they fit as closely as possible into the canal and against the walls of the prepared canal apically. In this way, Gutta Percha Points are used in combination with root canal paste sealers in conventional root canal therapy.

Traditionally, Silver Points (i.e. points formed from metallic silver) have also been used in dentistry as an alternative to Gutta Percha Points. Compared to Gutta Percha Points, Silver Points are more accurately calibrated, and can be more readily and rapidly sterilised. Also because they are more rigid, especially in the finer sizes, they are easier and quicker to use that Gutta Percha Points in filling narrow root canals.

On the other hand Gutta Percha Points, due to their greater flexibility, can be compacted closely against the root canal walls, thus providing effective sealing even in those apical parts of the prepared canal that deviate from a circular outline. Where the entire length of the root canal is filled with Gutta Percha Points, excess Gutta Percha in the pulp chamber can be removed with a hot instrument. Should removal of some of the root filling be necessary to provide room for a post, Gutta Percha is also easy to cut away. This will normally happen within several days or weeks of the Gutta Percha-based treatment. Due to the fact that a lot of root-filled teeth, even those that appear to have been successfully treated can subsequently fail in the course of months or years, the ease of removal of a root filling is also a very important material property.

The general technique of Gutta Percha Points use is as follows: A suitable point is selected and moulded to the apical end of the prepared canal. The apical 4 to 5 mm of the point are cut off and attached at its coronal end to a warmed root canal condenser. A suitable marker previously placed on the condenser is adjusted so that its distance to the tip of the point corresponds to the working length. The Gutta Percha Point is then carefully coated with cement and carried to the apical limit on the condenser. Gentle pressure is exerted in the apical direction to ensure good adaptation. The condenser is detached from the Gutta Percha Point by swinging it from side to side, at the same time as apical pressure is exerted. The remainder of the canal is obliterated with some "temporary" cement, which later may be readily removed in the event of a post construction, if necessary. A hazard of this method is that a section of the Gutta Percha Point may become detached from the condenser before the apical limit is reached.

The problems associated with the state-of-the-art dental endodontic thermoplastic materials, especially those used in the endodontic field, typified by Gutta Percha, are two-fold: Firstly, those materials lack bioactivity and do not adhere to tooth material. However, since the majority of root-canal obturation is carried out with root canal sealers in combination with the pre-formed Gutta Percha Points, the sealers contribute more to the tissues response to the restored root canal than the Gutta Percha Point. This leads to the second problem - the use of sealers and their lack of interaction to Gutta Percha Points.

Root canal sealers are used in conjunction with Gutta Percha Points in an attempt to produce an apical seal that prevents the ingress of fluids to the restored canal. Root canal sealers are also useful in creating the necessary adaptation to the lateral dentine wall of the root canal. Many of these sealers can also be used alone to fill the root-canals and have been developed specifically for use in this way. While Gutta Percha, which is several decades old, is still the most commonly used solid pre-formed indirect root-canal material used, several developments have since been made in the field of root canal sealers. At the present, there are dozens of root canal sealers available commercially, based on a number of different chemistries.

Scientific reports, many as early as the 1950s, detail the compositions of the earliest still-in-use root canal sealers. More recently root canal sealers have been prepared from diacrylates (WO 2002/13767) or bisacrylamide (WO 2002/137678). These root canal sealers exhibit a number of advantages such as low solubility and good sealing properties. However, the use of low molecular weight amines results in a serious disadvantage, namely the cytotoxic effects resulting from the amines being dissolved out of the root canal sealer which are frequently observed. A further addition to the class of root canal sealers are those which contain swellable hydrophilic acrylates and methacrylates such as HEMA (US Pat. No. 3,925 895). However, it is well known that small hydrophilic acrylate monomers such as HEMA have a poor polymerisation conversion and elicit an adverse (cytotoxic) response from the adjacent tissue.

Compounding this issue of most of the root canal sealers used in Gutta Percha/sealer root-canal restorations, is the fact that these sealers do not adhere to Gutta Percha, nor to the root canal wall. It is important that the root-canals restored with Gutta Percha Points/root canal sealer combination do not have such defect between the two restorative materials for two main reasons:
i) the defect, especially if it extends to the apical seal, may compromise the seal of the restored root canal to ingress of fluids from below, possibly leading to infection; and
ii) the defect will definitely compromise the radiopacity of the restoration and hence compromise any post operative diagnoses that may need to be carried out.

Therefore, the state of affairs at the moment in many routine restorations of root canals is Gutta Percha Points used in combination with an increasingly diverse range of root canal sealers, all of which to some degree are dissimilar enough from Gutta Percha to introduce some compromise in the restored root canal, be it from the point of view of cytotoxicity or materials interaction. This is in contrast to the ideal scenario of a root canal restored defect-free with a bioactive material or bioactive materials that contour well with each other and with the lateral dentine walls and also creates a good apical seal.

Thus, the object of the present invention is to provide a new means for filling root canal systems, comprising a filler material and/or an indirect point. In particular, the present invention is to provide new means to restore a root canal.

Another object of the present invention is to provide a bioactive hydraulic material capable of interacting with dental tissue as well as with the commercially available bioactive hydraulic cements such as MTA, bioactive calcium phosphate glasses and calcium phosphate cements. This material would facilitate the possibility of having a total bioactive restoration of root canals. Further, the inventive bioactive hydraulic sealer paste should be capable of temporary flowability, e.g. down narrow tooth canals to the apical tooth limit. The new material is to provide bioactive, anti-bacterial Points and Paste that bond to each other and to the dentinal walls.

Another object of the invention is to overcome the deficiency typified by pre-formed Points such as Gutta Percha Points of inadequate sealing property, by formulating Hydraulic Points that could undergo partial plastic deformation, partial surface dissolution, minimal expansion to effect rigid lateral and apical seal.

Optionally, non-hydraulic fillers and/or miscellaneous additives aimed at imparting specific cement properties may also be added to the composition of the present invention.

### SUMMARY OF THE INVENTION

In order to solve the issue of inadequate interaction between the root-canal points (Gutta Percha Points) and sealers, the present inventors recognised that the state-of-the-art and chemistries of indirect points and root canal sealers should be brought together to facilitate the ideal scenario for root canal restoration described in the preceding. As a consequence, the present inventors found that materials comprising a hydraulic cement can be advantageously used to pre-form Hydraulic Points, which can be used in a similar manner as Gutta Percha Points. Moreover, the same material comprising a hydraulic cement can be used as a sealer, either together with the Hydraulic Points or without. Thus, the present invention provides a generic formulation that can be used to fabricate:
i) Hydraulic Points to be used in a similar manner as Gutta Percha Points, and ii) Hydraulic pastes for use as sealers, with or without these Hydraulic Points.

Surprisingly, it was found that the objects of the present invention are advantageously reached with a generic Hydraulic Organic Matrix Material (HOMM) composition comprising:
a) at least one hydraulic cement powder, and
b) at least one organic matrix comprising an oil and/or fat and/or a thermoplastic material.

The HOMM composition of the present invention can be used both as i) a Hydraulic Point and ii) a Hydraulic Paste.

Advantageously, the inventive HOMM material is bioactive and capable of interacting with dental tissue as well as with the commercially available bioactive hydraulic cements such as MTA, Bioactive calcium phosphate glasses and calcium phosphate cements. This facilitates the possibility of having a total bioactive restoration of root canals.

Further, the inventive Hydraulic Points formed from the inventive HOMM material advantageously overcome the deficiency typified by other pre-formed Points such as Gutta Percha Points having inadequate sealing properties, because the inventive Hydraulic Points could undergo partial plastic deformation, partial surface dissolution, and minimal expansion to effect rigid lateral and apical seal.

Moreover, the inventive bioactive hydraulic sealer pastes are capable of temporary flowability, e.g. down narrow tooth canals to the apical tooth limit, due to the action of moderate energy, e.g. through the heat generated by the collision of the HOMM while being titurated in a dental capsule mixing machine at speeds typically in excess of 4000 revs/min. This heat is sufficient to temporarily raise the temperature of the HOMM to above its slip melting temperature and cause the temporary flowability.

Optionally, additives, such as e.g. non-hydraulic fillers, fillers having radiopacity and/or miscellaneous additives aimed at imparting specific cement properties, may also be advantageously added to the composition of the present invention in order to impart the inventive composition with the desired property.

By judicious choice of the organic matrix material, in particular the choice of the oil and/or fat and/or thermoplastic material(s), as well as the weight content thereof, both the inventive sealer paste and Hydraulic Point could be provided with various hardness, strengths and flexibilities.

### SHORT DESCRIPTION OF THE FIGURES

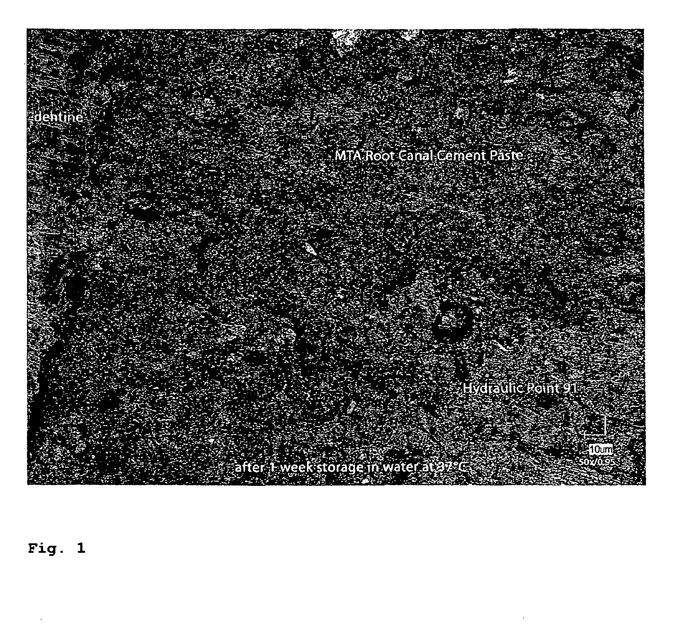

Figure 1 is a Laser Scanning microscopic photograph (Keyence VK-9710 Laser Scanning Microscope with magnification of x50) depicting a bovine tooth restored using the inventive hydraulic paste and an inventive hydraulic point (Hydraulic Point 91) after storage in water at 37°C for 1 week, showing the good adaptation of the paste to the dentinal wall. In Figure 1, the dentine wall is visible as uniform light grey area at the left end, the hydraulic point as light grey structure in the bottom right corner, and the set hydraulic paste as darker area in between.

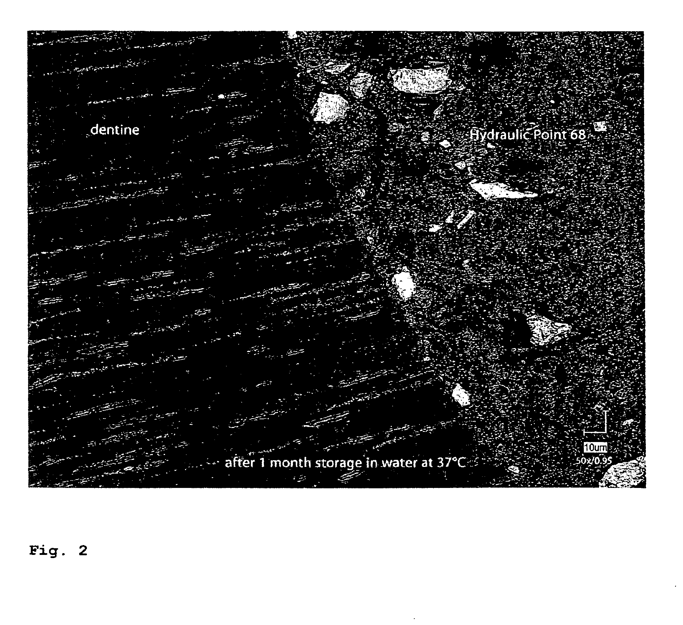

Figure 2 is a Laser Scanning microscopic photograph (Keyence VK-9710 Laser Scanning Microscope with magnification of x50) depicting a bovine tooth restored using the inventive hydraulic paste and an inventive hydraulic point (Hydraulic Point 68) after storage in water at 37°C for 1 month, showing the good adaptation of the paste to the dentinal wall. In Figure 2, the dentine wall is visible as light grey area on the left side of the figure, and the hydraulic point as dark grey matter on the right side.

### DETAILED DESCRIPTION OF THE INVENTION

The present invention provides a novel Hydraulic Organic Matrix Material (HOMM) composition comprising:
a) at least one hydraulic cement powder, and
b) at least one organic matrix comprising an oil and/or fat and/or a thermoplastic material.

The material of the invention is bioactive and capable of interacting with and adhering to dental tissue as well as the commercially available bioactive hydraulic cements such as MTA. The composition can be provided in various formulations from single soft pastes to rigid semi-hard to hard pre-formed Points. In addition, the composition can also be made very readily flowable, e.g. down narrow tooth canals to the apical tooth limit. Therefore, the inventive composition can advantageously be used in numerous dental and especially dental endodontic applications.

The Hydraulic Pastes cements of this invention are also potentially suited as hardening wound dressing materials and also as water-hardening endodontic or temporary restorative cements. In the context of this invention, a paste is defined as a soft material (typically with shore A less than 60) that is readily deformable (smeared) without it losing its cohesion. Points, however, are harder and rigid, typically with a Shore A greater than 60 and do not readily lend themselves to such deformation without loss of cohesion.

Finally, the pre-formed rigid Hydraulic Points, exhibiting a number of significant advantages over Gutta Percha (such as bioactivity-high pH, adhesion, etc), are a viable alternative to Gutta Percha and could in future become the material of choice in selected endodontic treatments.

The pre-formed Hydraulic Points is just one example of pre-formed shapes that can be fabricated from the inventive HOMM. In the examples provided in this invention, the Points have been rolled out at temperatures just above their slip melt temperatures and then allowed to cool down slowly. Pre-formed shapes are most typically in dimensions between 0,5mm - 50mm. Pre-formed shapes can be Points or other shapes, such as granules.

This invention is further characterised by the fact that the inventive compositions can be pre-fabricated into Hydraulic Points, e.g. for use as Gutta Percha-type endodontic points; or formulated into Hydraulic sealer pastes, e.g. for use as endondontic root canal sealers or prophylaxis pastes or restorative materials. In particular, it was found that by the judicious choice of Organic Matrix Material, in particular the choice and weight content of thermoplastic, oil or fat materials, both sealer paste(s) and Hydraulic Points with various hardness, strengths and flexibilities could be produced.

### DEFINITIONS OF MATERIALS USED IN THE INVENTION

### HYDRAULIC CEMENTS

In the sense of this invention, Hydraulic Cements are cements where water is the main reactant that participates in the hardening of a typically particulate material to an alkaline mass. Non-alkaline Hydraulic cements have been used in dentistry for several decades, in particular as gypsum-based dental plaster, stone and casting investment materials and also as temporary restorative formulations. More recently, within the last 20 years, a number of bioactive (and alkaline) hydraulic cements have been developed and commercially introduced into the dental field. These include the Mineral Trioxide Aggregate (MTA) - a Portland cement-type material; bioactive alkaline soda-containing calcium phosphate glasses, such as the Bioglass®; and the so-called Calcium Phosphate cements, based on crystalline phosphate species.

### HYDRAULIC CEMENTS - MTA Cements

Within the last 20 years the Mineral Trioxide Aggregates (MTA) cements have been developed and commercially launched. Many publications, in particular by the M. Torabinejad, describe these materials. Notable publications by this author include "Physical and chemical properties of a new root end filling material", Journal of Endodontics, vol. 21, pp 349-53 (1995); "Tissue ration to implanted root-end filling materials in the tibia and mandible of guinea pigs", Journal of Endodontics, vol. 23, pp 225-283 (1997); "investigation of Mineral Trioxide aggregate for root-end filling in dogs, Journal of Endodontics, vol. 22, pp 575-78 (1995); "Cytotoxicity of four root-end filling materials, Journal of Endodontics, vol. 21, pp 4899-92 (1995); "Dye leakage of four root end filling materials: effect of blood contamination, Journal of Endodontics, vol. 20, pp 159-63 (19945); and "Sealing ability of mineral trioxide aggregate when used as a root-end filling material, Journal of Endodontics, vol. 19, pp 591-95 (1993).

MTA is usually presented as a powder that is mixed with water to form a cementitious mass that becomes resistant to water dissolution within 1 to 6 hours. The major constituents of the powder are calcium oxide, aluminium oxide, silicon dioxide and iron oxide. The Trioxides responsible for the water-setting reaction of the MTA cements are: i) 2CaO-SiO₂ = Ca₂SiO₄; ii) 3CaO·SiO₂ = Ca₃SiO₅; and iii) 3CaO· Al₂O₃ = Ca₃Al₂O₆. Although the complete hardening reaction of the MTA (and Portland) cements are complex, the basics steps are believed to involve first the reaction of Ca₂SiO₄ and Ca₃SiO₅ with water to form principally the cementitious tricalcium silicate hydrate. Excess precipitated Ca²⁺ ions into aqueous solution form Ca (OH) ₂, the species responsible for the high alkalinity of the MTA cements (pH 10-13 for 3 minute-old cements). The clinical advantages exhibited by MTA cements have been related to this their in-situ produced Ca(OH) ₂. These Ca(OH) ₂-based properties have been reported: i) to be antimicrobial; ii) to be anti-inflammatory; iii) to inhibit dental tissue resumption; and iv)with the aid of the phosphate of body/oral fluids (including blood), to form calcium apatite hard tissue.

MTA are hydraulic cements that contain at least one of the mineral trioxide Ca₃Al₂O₆, Ca₂SiO₄, and Ca₃SiO₅, but which usually contain up to three or more major phases and some other minor ones as well. In Table 1 summarises several chemical composition of MTA cement powders from US2009/0314181 A1 of 2009 by Primus.

**Table 1 The Chemical Composition of selected MTA Cement Powders**

| **Component** | US Pat 5,415,547 **MTA** | **Colton Fast set Portland Cement** | US2009/0314181 A1 **MTA** |
|---|---|---|---|
| Calcium oxide | 65,00 | 64,2 | 68,9 |
| Silicon dioxide | 21,00 | 20,8 | 25,2 |
| Iron oxide | 5,00 | 4,3 | 0,3 |
| Aluminium oxide | 4,00 | 3,9 | 2,0 |
| Magnesium oxide | 2,00 | 3,2 | 0,6 |
| Sulphates | 2,50 | 2,6 | 2,2 |

MTA cements are usually provided in powder form, i.e. having particle sizes of D₉₀ typically less than 500 microns, but more usually with D₉₀ of between 20 and 200 microns.

### HYDRAULIC CEMENTS - Bioactive Calcium Phosphate Glasses

Bioactive glasses such as the BioGlass® cements are water-setting soda-calcium phospho-aluminosilicates, similar to the MTA cements in their use and properties. They are SiO₂ Na₂O, CaO, P₂O₅ hydraulic cement glass powders with the ability to produce a bioactive surface of hydroxyapatite within minutes of water activation (Hench, L. and Andersson, O, "Bioactive Glasses" World Scientific Publishing Co Pte Ltd, Singapore, 1993 pp 41-63). They find limited use in the dental Endodontic field as alkaline (anti-bacterial) cements.

Bioactive calcium phosphate glasses are hydraulic cement powders. They can contain silicates as well as phosphate species. A typical composition range for the Bioglass®, a bioactive calcium phosphate glass is given below in Table 2.

**Table 2. Typical Chemical Composition of a BioGlass-type Powder**

| **Constsituents** | **Weight percent** |
|---|---|
| Silicon dioxide | 40 - 52 |
| Calcium dioxide | 10 - 50 |
| Sodium oxide | 10 - 35 |
| Phosphorus pentoxide | 2 - 8 |
| Calcium fluoride | 0 - 2,5 |
| Boron oxide | 0 - 10 |

Bioactive calcium phosphate glasses are usually provided in powder form, i.e. having particle sizes of of D₉₀ typically less than 500 microns, but more usually with D₉₀ of between 20 and 200 microns.

### HYDRAULIC CEMENTS - Calcium Phosphate Cements

Calcium Phosphate cements are based on the crystalline phosphates of the type dibasic calcium phosphate, α-tribasic calcium phosphate, tetrabasic calcium phosphate, etc which are mixed with water or aqueous sodium phosphates to form cements. They are used primarily as biomedical cements for the reinforcement of natural bone and also as maxillofacial cements.

A hydraulic calcium phosphate cement preferably comprises crystalline calcium phosphate and/or crystalline calcium silicate particles. The particles preferably have particle sizes of D₉₀ typically less than 500 microns, but more usually with D₉₀ of between 20 and 200 microns.

There have been several attempts by various workers to improve the properties of this class of hydraulic cements. These include works reported in the following patent publications:
Hirano, M; Takeuchi, H and Asaoka, N in EP1364925 A1 of 2001 in which between 0.03 and 2 wt percent sodium pyrophosphate was added to compositions of calcium phosphates in a bid to improve the hardening strength of Calcium Phosphate Cements.

Takenori Sawamura and Hattori Masahiko Okuyama in the US patent 5993535 of 1999 in which polysaccharides was added compositions of calcium phosphates in a bid to reduce their setting times.

Takenori Sawamura and Masateru Hattori in the US patent 5980625 of 1999 in which pectin was added to compositions of calcium phosphates in a bid to reduce their setting times.

Calcium Phosphate cements are hydraulic cements that contain crystalline calcium phosphates species. There are a number of Calcium phosphate cements available and they contain a multitude of phosphate species, including (TTCP) tetra calcium phosphate Ca₄(PO₄)₂O; (β-TCP) β-Ca₃(PO₄)₂; α-TP; (MCP) mono calcium phosphate; Hydroxyapatite Ca₅(PO₄)₃OH; DCP dicalcium phosphate anhydrous CaHPO₄; (DCPD) dicalcium phosphate dehydrate CaHPO₄.2H₂O; MgHPO₄ and very small amounts of NaH₂PO₄; Na₂HPO_{4,} etc. Cacium phosphate cements can also contain non-phosphate species, such as CaCO₃; Ca(OH)₂; CaSO₄; MgSO₄; Mg(OH)₂

Calcium Phosphate cements are usually provided in powder form, i.e. having particle sizes of D₉₀ typically less than 500 microns, but more usually with D₉₀ of between 20 and 200 microns.

### ORGANIC MATRIX - Thermoplastic, Oils and Fats

The Organic Matrix comprises an oil and/or fat or a thermoplastic, or a combination thereof. It can be polymeric or non-polymeric, synthetic or naturally-occurring. At room temperature, it can be a liquid or a solid.

In the sense of this invention, thermoplastics are defined as materials that undergo significant change in flow properties upon application of heat. Preferably, thermoplastics used in the present invention melt within the temperature range of ambient temperature (i.e. 20°C) to a few degrees below 100°C (e.g. 95°C). Therefore, in this context thermoplastics can be polymeric, e.g. synthetic polymers based on polyalkanes, or polyethyleneglycol (PET), or naturally-occurring polymers based on isoprene and related isomers; or non-polymeric wax esters e.g. natural vegetable waxes (Carnauba wax) and animal-based waxes (shellack wax). Thermoplastics can also be non-synthetic resins such as colophony resin, kauri resins, etc. Resins are glycerides obtained from the reaction of mixtures of fatty acids while waxes or wax esters are obtained from single fatty acids.

Oils or fats, on the other hand, are the reaction product of fatty acids and alcohols, in particular, between long chain fatty acids and long chain alcohols, i.e. esters. Examples of oils comprise mineral oils or naturally-occurring oils, including volatile oils/essences. The naturally-occurring oils are triglycerides obtained from the reaction of long chain fatty acids and glycerine. Of course, synthetic oils can be non-glycerides, di glycerides and mono glycerides. In our classification Fats are Oils with higher melting points due to their longer chain lengths in comparison to oils.

### Oils/fats

Oils are ester reaction products of fatty acids and alcohols. The longer the chain length of the fatty acid the higher the melting temperature of the resulting ester. Esters that are solid at room temperature are usually referred to as Fats and those that are liquid at room temperature are usually referred to as Oils. A more detailed description of oils and fats can be found in the standard Chemistry textbook "Organic Chemistry" by Douglas C. Necker and Michael P. Doyle, Wiley poublishing company, pages 446 -450. Examples of oils and Fats include: 2-ethylhexl palmitate, C16 - C18 Trigylcerides based on vegetable oils, C16 - C18 Trigylcerides, Shear butter, Oleic acid oleyl ester, polyol caprylic/capric fatty acid ester (PEG-6 caprylic/capric glycerides), 2-ethyl hexyl palmitate/stearate, Lauric acid hexylester, polol coconut fatty acid ester, coconut fatty acid isopropanolamide, ethylene glycol distearate, Glyceryl stearate, cetaaryl alcohol, cetyl palmitate, cocoglycerides, fatty alcohol, fatty acid ester, polyoxythylene glycerine monostearate, PEG-Glyceryl stearate, glyceryl monostearate, hydrogenated castor oil, fatty acid polyglycol ester, fatty alcohol ethoxylates, polyglycerine poly-12-hydroxystearic acid ester, cetearyl alcohol, glyceryl stearate, glycerine, certyl palmitate, cetyl stearyl polyglucoside, glyceryl polyacrylate, myristic acid isopropylester, glycol distearate, coco glucoside, glyceryl oleate, macadamia oil, mineral oil, propylene glycol dicaprylate, jojoba oil, resin oil, Neat foot oil, Canada balsam oil, oleo resin, etc.

### Thermoplastics

Thermoplastics are polymers that can be melted and undergo significant change in rheological properties upon the application of heat. Thermoplastics can be synthetic or naturally-occurring and can be further sub-classified as crystalline or non-crystalline. Typically, Thermoplastics are solids at ambient temperatures and liquids at temperatures approaching 100°C. Polymeric waxes and wax esters are subsets of Thermoplastics. Typically, at 20°C they are kneadable in consistence and can be plastic or brittle, amorphous or crystalline, translucent or opaque. At temperatures over 40°C, without supplementary additives, they will begin to melt and become very fluid. There exists another class of Thermoplastics that are non waxing synthetic or natural products, typically termed resins. These are distinct from waxes in that melt at relatively higher temperatures of between 50°C and 90°C but also up to 200°C.

Typical examples of thermoplastics useful in this invention include: waxes of animal origin or vegetable waxes such as Euphorbia Cerifera (Candelilla) wax and Candelilla Cera, Carnauba wax, Gutta Percha wax, Esparto wax, Guaruma wax, Cork wax, jojoba wax, etc. Typically examples of non waxing Thermoplastics useful in this invention also include Resins such as colophony resin, staybiolite resin, Kauri resin, etc.

Further on, the inventive formulations may contain:

### Optional Additives

Additives can be added to the cement for a number of reasons. Additives can be additional fillers. If desired, additives can be plasticizers, indicators (e.g. for monitoring the reaction by pH changes, etc.), dyes, pigments, viscosity modifiers, wetting agents, chelating agents (e.g. reaction speed modifiers-chelating the released Ca²⁺), surfactants, buffering agents, stabilizers, and other similar ingredients that will be apparent to those skilled in the art. Additionally, medicaments or other therapeutic substances can be optionally added to the dental compositions.

Examples of suitable non-oil type additives includes, cetaaryl alcohol, fatty alcohol with C12 - C18, palmitic acid, stearic acid, modified alcohol polyglycol ether, etc.

Other miscellaneous additives of this invention include whitening agents, anticaries agents (e.g., xylitol), remineralizing agents (e.g., calcium phosphate compounds), enzymes, breath fresheners, anesthetics, clotting agents, acid neutralizers, chemotherapeutic agents, immune response modifiers, thixotropes, polyols, anti-inflammatory agents, antimicrobial agents, antifungal agents, agents for treating xerostomia, desensitizers, and the like, of the type often used in dental compositions. Combination of any of the above additives may also be employed. The selection and amount of any one such additive can be selected by one of skill in the art to accomplish the desired result without undue experimentation.

Further compositions of the invention may contain:

### Optional Non-hydraulic Fillers

These fillers are usually substantially inert in the water-based hydration setting reaction of the cement. The fillers can be oxyfluorides (materials that contain oxygen and fluorine) such as ytterbium fluoride, strontium fluoride, calcium fluoride, etc. The fillers can also be non-fluoride containing and can additionally chosen from rare earth metal cations to impart improved radioacity on the inventive cements. Suitable examples are the divalent cations of Ca²⁺, Sr²⁺, Ba²⁺, Ce²⁺, TiO²⁺, ZrO²⁺, Fe²⁺, Co²⁺, Cu²⁺, Zn²⁺ or the trivalent cations of Sc³⁺, Y³⁺, La³⁺, Yb³⁺, Cr³⁺, Mo³⁺, W³⁺, Fe³⁺, Ru³⁺, Os³⁺, Au³⁺, Al³⁺, Ga³⁺, Bi³⁺, In³⁺ in combination with anions such as O²⁻, [CO₃]²⁻, Cl⁻, [SO₄]²⁻, [PO₄]³⁻, [HPO₄]²⁻, [H₂PO₄]⁻, [HPO₂]²⁻, [H₂PO₃]⁻, etc. Other suitable fillers include acid-reactive glasses, inert dental composite-type glasses, glass-ceramics, ceramics and minerals, especially silicate clay-type ones, such as bentonite, montmorillonite, etc. These non-hydraulic fillers could have particle size similar to the HOMM bioactive powders, i.e. D₉₀ typically less than 500 microns, but more usually with D₉₀ of between 20 and 200 microns. They could also be much bigger, i.e. granules with D90 typically between 200 microns and 1mm

Most preferred of the fillers are materials with high radiopacity and very low oral solubility such as bismuth oxide, calcium tungstate, ytterbium fluoride, lanthanum oxide, barium sulphate, etc.

Especially preferred are fillers having a radiopacity of greater than 150% Al measured according to the dental ISO Standard ISO4049, which standardises the radiopacity of 2mm-thick specimens to the radiopacity of pure Al metal.

Preferably, the inventive composition may comprise from 20 to 60 weigth % based on the total content of the composition.

A hydraulic paste according to the present invention is a composition comprising at least one hydraulic cement and the at least one organic matrix in a ratio of from 1:99 to 99:1, but preferably between 30:70 to 70:30.

The inventive hydraulic paste is soft and has both formability and formability at room temperature.

The inventive paste preferably exhibits a slip melting point or "slip point" temperature of from 40 to 80°C, preferably between 50 to 70°C.

The slip point melting temperature is determined by casting a 10mm column of solid in a glass tube with an internal diameter of 1 mm and a length of 80 mm, and then immersing it in a temperature-controlled water bath. The slip point is the temperature at which the column of the solid begins to rise in the tube due to buoyancy, and because the outside surface of the solid is molten. This property, the slip point, is a particularly popular method of characterising fats and waxes, as they tend to be mixtures of compounds with a range of molecular masses, without well-defined melting point.

Preferably, the inventive hydraulic paste exhibits a Shore A hardness value of 50 or less, preferably of from 5 to 15. According to the invention, the Shore A hardness value means the Shore A hardness value as measured by the Shore A hardness equipment comprising a needle and a load force according to ISO R868, DIN 53505.

The hydraulic paste of the present invention may be prepared by mixing, with the aid of an organic solvent that is later expelled under heat, the Organic Matrix and the bioactive hydraulic powder.

Preferably, the inventive hydraulic paste is provided in a dental capsule that can be readily mixed in a standard dental apparatus, e.g. an amalgamator mixing device.

Upon mixing, the hydraulic paste is provided with an initial flowable consistency through the increase in its temperature to above its slip melting temperature by the action of the excessive mixing speed of tituration. This lasts typically for for 1 to 2 minutes, while the temperature of the Paste returns to below its slip melting point, thus enabling the paste to be introduced into a prepared dental cavity.

Upon contact with water, the inventive hydraulic paste usually achieves clinical sets within 10 to 120 minutes, resulting in a cement that binds well to dentine. Further water uptake and hardness development continues for the next 24 hours.

A hydraulic Point according to the present invention is an endodontic point. Endodontic points known in the art comprise standard endodontic points made of Gutta Percha or the like.

The inventive hydraulic point may have the dimensions of standard endodontic points. Preferably, the hydraulic point has the following typical dimensions/ranges length (10 to 30mm) /width (0.5 to 2mm) /tapering to an "apex" of (0.2 to 1mm) etc.

The hydraulic point of the present invention may be prepared by mixing, with the aid of an organic solvent that is later expelled under heat, the Organic Matrix and the bioactive hydraulic powder.

Preferably, the hydraulic point exhibits a slip point melting temperature of from 50 to 70°C.

Thus, the hydraulic point may advantageously be removed using a hot instrument, such as a hot condenser tip, or the like.

Preferably, the inventive hydraulic point exhibits a Shore A hardness value of 95 or less, preferably of from 40 to 80.

Preferably, the hydraulic point may be used together with the hydraulic paste to fill a prepared dental cavity, such as a root canal, or the like.

There are a number of advantages inherent in the use of the inventive materials: i) the materials(paste and Points) are bioactive, slightly plastically deformable and with high pH, providing for a antibacterial barrier; and ii) the materials (paste and Points), having very similar formulation and chemistries, interact to form a seamless entity with very similar thermal and water expansions properties. This is in contrast to the state-of-the art of the use of Gutta Percha Points with the commercially available resin-based sealants.

### EXAMPLES

The compositions of the invention were prepared using two basic methods: i) the blending of the thermoplastic polymer, oil and/or fat with the hydraulic cement powder using a water-free organic solvent, such as ethyl acetate, chloroform, etc; and then heating the mixture to 105°C while stirring to achieve intimate mixture of the components and to expel the solvent; and ii) the blending of the thermoplastic polymer with the hydraulic cement powder in the absence of a solvent and heating the mixture to 105°C while stirring to achieve intimate mixture of the components.

The present invention is illustrated in the following examples using the hydraulic cement powder MTA-Universal Direct Cap available from Cumdente GmbH, Tübingen, Germany.

| **Properties of MTA Universal** | **Values** |
|---|---|
| Particle size profile | D10 = 1,5mu; D50 = 6mu; D99 =26mu |
| Water content of powder | 1% |
| pH upon mixing with water | 12 |

In addition the following 3 ingredients were used:
Ingredient 1: A mixture of 7 parts of (C14-C20 triglycerides); 3 parts of (hydrogenated vegetable oil) and 1 part of the thermoplastic candelilla Cera
Ingredient 2: hydrogenated castor oil
Ingredient 3: ytterbium fluoride powder with particle size D90 of 10 microns.

The slip melting temperature was measured using as described in the preceding section.The pH was measured using a universal pH paper.

The flowable phase of the paste was determined by measuring the disc diameter of the 0,25g of cement that had been subjected to a load of 5kg for 30s. The more flowable the cement is, the greater is the spread of the cement.

The Shore A hardness of the points was measured as described in the preceding section in accordance with ISO R868 (DIN 53505)

### Example 1

### Preparation of MTA Root Canal Cement Paste

To 10g of MTA-Universal Direct Cap powder were added 40g of ethyl acetate, 10g of a 7:3:1 mixture of (C14-C20 triglycerides: hydrogenated vegetable oil: candelilla Cera) and 100g of ytterbium fluoride. The mixture was heated up to 105°C while stirring to achieve an intimate mixture of the constituents, this being noticeable when the mixture lost its initial grainy consistency and exhibited a uniform surface gloss. Thereafter, the mixture was left overnight at this 105°C in order to expel the ethyl acetate, noticeable when the mixture no longer exuded the characteristic acetate odour. Upon cooling the resulting paste was soft (i.e. easily deformable without loss of cohesion) and slightly flowable at temperatures above 50°C.

### Paste Properties

Slip melting temperature = 50°C

pH = 11

Mixing time in Amalgamator at 4300 rpm = 10s

Working Time (flowable phase of the paste) after mix = approx. 2 minutes

Flow Properties of the Root Canal Paste upon Tituration

| Time from onset of mix | Consistency (flow character), in mm Spread of 0,25g of cement after load of 5kg for 30s |
|---|---|
| Without amalgamator mix | 25 mm |
| 1 min after mix | 38 mm |
| 2 mins after mix | 36 mm |
| 3 mins after mix | 28 mm |
| 4 mins after mix | 25 mm |

The ability to impart flow on the paste, in this instance due to the energy of the Amalgamator, can be important for the clinical efficacy of the material. It enables the paste to the introduced down a narrow chamber, e.g. down the root canal to the root apex within the working (flowable) time of the paste. Once in the final restoration position, subsequent water-hardening reaction of the paste will follow. Good adaptation to dentine substrate can also be enhanced by this flow. This is shown in Figure 1 of the inventive Root Canal Cement Paste in a bovine tooth restoration.

The speed at which Titurators operate (e.g. >4000 revs/min) can be excessive to what is required to just achieve a thorough mix. The high speed at which the mixing materials collide converts to energy and thence an increase in temperature of the material being mixed. With HOMM with relatively low melt temperatures (e.g. 40°C - 60°C), this increase in temperature is enough to push the material over its melting point and hence make it much more flowable than it hitherto was at ambient temperatures. This state of high flow is obviously transient, i.e. lasts only while the material is above the melting temperature. Furthermore, tituration (at high speeds) is only one way to achieve a temporary flow in a thermoplastic material. Others include heat and ultrasound, etc.

This inventive Root Canal cement Paste overcomes some of the noted (state-of-the art background information) deficiencies of Gutta-percha. It also overcomes the deficiencies that are limiting the use of the current commercial alkaline bioactive cements (MTA, etc) in more dental endodontic applications.

### Example 2

### Preparation of Hydraulic Point 91

To 10g of MTA-Universal Direct Cap powder were added 40g of ethyl acetate, 10g of hydrogenated castor oil and 10g of ytterbium fluoride. The mixture was heated up to 105°C while stirring to achieve an intimate mixture of the constituents and then thereafter to expel the ethyl acetate. Upon cooling the resulting mass was formed into Points while at temperatures just above its slip point.

### Point Properties

Slip melting temperature = 90°C

pH = 11

Shore A hardness = 91

### Example 3

### Preparation of Hydraulic Point 74

To 10g of MTA-Universal Direct Cap powder were added 40g of ethyl acetate, 8g of hydrogenated castor oil, 2g of 7:3:1 mixture of (C14-C20 triglycerides: hydrogenated vegetable oil and candelilla Cera) and 10g of ytterbium fluoride. The mixture was heated up to 105°C while stirring to achieve an intimate mixture of the constituents and then thereafter to expel the ethyl acetate. Upon cooling the resulting mass was formed into Points.

### Point Properties

Slip melting temperature = 70°C

pH = 11

Shore A hardness = 74

### Inventive Example 4 - Hydraulic Point 68

To 10g of MTA-Universal Direct Cap powder were added 40g of ethyl acetate, 5g of hydrogenated castor oil, 5g of a 7:3:1 mixture of (C14-C20 triglycerides: hydrogenated vegetable oil and candelilla Cera) and 10g of ytterbium fluoride. The mixture was heated up to 105°C while stirring to achieve an intimate mixture of the constituents and then thereafter to expel the ethyl acetate. Upon cooling the resulting mass was formed into Points.

### Point Properties

Slip melting temperature = 65°C

pH = 11

Shore A hardness= 68

Thus, three Hydraulic Points with very differing hardness were prepared. The ability to formulate Hydraulic Points with varying Hardness demonstrates the versatility of this invention.

### Example 5

### Efficacy as endodontic and restorative materials

In order to demostrate the efficacy as endodontic and restorative materials in dental applications, in-vitro studies were carried out in bovine teeth. In these experiments, the Hydraulic Points prepared in Examples 2 to 4 were used in combination with the Hydraulic Paste prepared in Example 1.

Clean bovine teeth prepared with root canal cavities were restored with the MTA Root Canal Cement Paste (Example 1) spread around the Hydraulic Point 91 (Example 2). Figure 1 shows the good adaptation of the MTA Root canal cement Paste (Example 1) to the dentinal walls and to the Hydraulic Point 91 (Example 2). This means that in a root canal restoration with these two materials there will be seamless transition between all the materials involved in the restoration - MTA Point (e.g. in Examples 2 to 4) - MTA Root canal cement paste - dentine. In such a root canal restoration the Hydraulic Point 91, with a similar rigidity as Silver Points, or the Hydraulic Point 74 or the Hydraulic Point 68 or could be used according to the hardness preference of the dental practitioner.

The Hydraulic Point 74 and Hydraulic Point 68 are both similar in hardness to Gutta-Percha (hardness 70) and from the standpoint of hardness can be used in a similar manner to Gutta-Percha in endodontic treatment work. Such a use of Hydraulic Point 68 is shown in Figure 2. Figure 2 shows the good adaptation of a Hydraulic Point to dentine. This good adaptation can be seen from the lack of gap at the interface of the two materials (Point and dentine. This is in contrast to Gutta-percha which is adapted to dentine only through the use of root canal sealers.

The good adaptation of the invented Hydraulic Points to dentine is a significant improvement on the state-of-the-art endodontic Points such as Gutta Percha. Added to the adaptation of the invented Hydraulic Points to the invented Pastes and the adaptation of the Pastes to the dentinal walls (Figure 1), with this invention, the present invention provides the means to restore a root canal with bioactive, anti-bacterial Points and Paste that bond to each other and to the dentinal walls.

The hardness of an inventive paste/inventive Hydraulic Points is as follows:

| HOMM | Shore A (hardness) | Approx. Slip melting temperature °C |
|---|---|---|
| Paste | 50 | 45 |
| Point 68 | 68 | 65 |
| Point 74 | 74 | 70 |
| Point 91 | 91 | 90 |

There are two preferred ways to formulate pastes or points, the pastes being "soft" and the points "hard":
i) The more thermoplastic content (i.e. the less oil/fat content) the Organic Matrix has, the more flexible is the resulting "Hydraulic Organic Matrix Material" (HOMM). In terms of composition, the transition from paste to point (non-paste) is not very sharp. Generally, pastes have thermoplastic content between 8 and 100 weight %, with respect to the Organic Matrix content. Conversely, points have thermoplastic content between 0 and 8 weight % (with respect to the Organic Matrix content) of greater than 92 weight %.

### Inventive Examples

| HOMM | 10% Thermoplastic - (7:3:1) Mixture | Castor oil |
|---|---|---|
| Paste | 10g | 0g |
| Point 68 | 5g | 5g |
| Point 74 | 2g | 8g |
| Point 91 | 0g | 10g |

ii) reduce the content of Organic Matrix in the formulation, i.e. increase the powder bioactive material content. Then all permutations of oils/fats and thermoplastic can be used to create both Pastes and Points.

According to a preferred embodiment, the Hydraulic Points undergo hydraulic reaction and harden slowly to reach apprx 90% of their final hardness over the first 24 hours of contact with water at 37°C. The hardness can be determined by Shore A hardness measurements.

The inventive pastes and/or the inventive points can, e.g., be used as follows: i) use of a paste in combination with a point; ii) use of a paste alone; and iii) use of a point alone. The use of the point alone especially at a narrow root, due to its partial plastic deformation, partial surface dissolution, and minimal expansion might be sufficient to effect rigid lateral and apical seal.

## Claims

1. Composition comprising:
a) at least one hydraulic cement, and
b) at least one organic matrix comprising an oil and/or fat and/or a thermoplastic material.

2. Compositions according to Claim 1, wherein the hydraulic cement is selected from the group consisting of a MTA cement, a calcium phosphate-type glass cement and a calcium phosphate cement.

3. Compositions according to Claim 1 or 2, wherein the hydraulic cement comprises Ca₃Al₂O₆, Ca₂SiO₄, and/or Ca₃SiO₅, or a combination thereof.

4. Compositions according to one of Claims 1 to 3, wherein the hydraulic cement comprises crystalline calcium phosphate and/or crystalline calcium silicate particles.

5. Compositions according to one of Claims 1 to 4, wherein the organic matrix comprises an oil comprising glycerol and one to three fatty acids.

6. Composition according to one of Claims 1 to 4, wherein the oil is a hydrophilic monoglyceride, diglyceride or triglyceride.

7. Compositions according to one of Claims 1 to 4, wherein the oil is an alkylester.

8. Compositions according to one of Claims 1 to 7, wherein the organic matrix comprises a thermoplastic polymeric wax or wax ester.

9. Composition according to Claim 7, wherein the thermoplastic polymeric wax or wax ester is selected from the group consisting of the vegetable wax cadelilla Cera, jojoba wax, and carnabau wax.

10. Composition according to one of Claims 1 to 9, wherein the organic matrix comprises a resin material.

11. Composition according to Claim 10, wherein the resin material is selected from the group consisting of oleo resin, calophony resin, staybiolite resin, and kauri resin.

12. Composition according to one of Claims 1 to 11, wherein the content of the organic matrix is from 10 to 90 weight % of the total content of the composition.

13. Composition according to one of Claims 1 to 12, wherein the content of the hydraulic cement powder is from 10 to 90 weight % of the total content of the composition.

14. Compositions according to one of Claims 1 to 13, further comprising at least one additive selected from an alcohol, an acid and a filler.

15. Composition according to Claim 14, wherein the filler is selected from the group consisting of Lanthanum oxide, ytterbium fluoride, and inert radiopaque glass with radiopacity of greater than 150% Al.

16. Compositions according to one of Claims 14 or 15, wherein the content of the additive is from 20 to 60 weight % of the total content of the composition.

17. Composition according to one of Claims 1 to 16, formulated into a hydraulic pastes for use as a prophylaxis paste or as an endodontic or restorative material.

18. Composition according to Claim 17, wherein the hydraulic paste has a slip point melting temperature of from 50 to 70°C.

19. Pre-shaped form or Endodontic point formed from the composition according to any one of claims 1 to 16.

20. Kit, comprising a composition according to one of Claims 17 or 18 and a pre-shaped form and/or endodontic point according to Claim 19.
